# EUROPEAN PATENT SPECIFICATION

(11) **EP 1 399 065 B1**
(45) Date of publication and mention of the grant of the patent: **05.10.2011**
(21) Application number: 02737433.9
(22) Date of filing: 07.06.2002
(51) Int. Cl.: A61B 5/15

(54) **SAMPLING DEVICES AND METHODS UTILIZING A STEPPED CAPILLARY PASSAGEWAY**
ENTNAHMEEINRICHTUNG UND VERFAHREN BEI ANWENDUNG EINES ABGESTUFTEN KAPILLARENDURCHGANGES
DISPOSITIFS D'ECHANTILLONNAGE ET PROCEDES D'UTILISATION D'UN PASSAGE CAPILLAIRE ETAGE

(30) Priority: 08.06.2001 US 296949 P; 29.08.2001 US 315873 P; 08.04.2002 US 370683 P
(43) Date of publication of application: 24.03.2004
(73) Proprietor: F.Hoffmann-La Roche AG, 4070 Basel (CH); Roche Diagnostics GmbH, 68305 Mannheim (DE)
(72) Inventor: ROE, Jeffrey, N., San Ramon, CA 94583 (US); ALLEN, John, J., Mendota Heights, MN 55118 (US)
(74) Representative: Jung, Michael
(86) International application number: PCT/US2002/018205
(87) International publication number: WO 2002/100277

(56) References cited:
- US-A- 5 951 492
- US-A- 6 048 352
- US-A- 6 099 484
- US-B1- 6 332 871

## Description

### Background of the Invention

### Field of the Invention

The present invention relates to the sampling of a bodily fluid obtained from an incision in the skin, and more particularly to acquiring the fluid by capillary action. The invention also may include the combination of such sampling devices and methods with incising, expressing, and/or testing systems.

### Description of the Prior Art

The acquisition and testing of bodily fluids is useful for many purposes, and continues to grow in importance for use in medical diagnosis and treatment, and in other diverse applications. In the medical field, it is desirable for lay operators to perform tests routinely, quickly and reproducibly outside of a laboratory setting, with rapid results and a readout of the resulting test information. Testing can be performed on various bodily fluids, and for certain applications is particularly related to the testing of blood and/or interstitial fluid. Such fluids can be tested for a variety of characteristics of the fluid, or analytes contained in the fluid, in order to identify a medical condition, determine therapeutic responses, assess the progress of treatment, and the like.

The testing of bodily fluids basically involves the steps of obtaining the fluid sample, transferring the sample to a test device, conducting a test on the fluid sample, and displaying the results. These steps are generally performed by a plurality of separate instruments or devices.

One method of acquiring the fluid sample involves inserting a hollow needle or syringe into a vein or artery in order to withdraw a blood sample. However, such direct vascular blood sampling can have several limitations, including pain, infection, and hematoma and other bleeding complications. In addition, direct vascular blood sampling is not suitable for repeating on a routine basis, can be extremely difficult and is not advised for patients to perform on themselves.

The other common technique for collecting a bodily fluid sample is to form an incision in the skin to bring the fluid to the skin surface. A lancet, knife or other cutting instrument is used to form the incision in the skin. The resulting blood or interstitial fluid specimen is then collected in a small tube or other container, or is placed directly in contact with a test strip. The fingertip is frequently used as the fluid source because it is highly vascularized and therefore produces a good quantity of blood. However, the fingertip also has a large concentration of nerve endings, and lancing the fingertip can therefore be painful. Alternate sampling sites, such as the palm of the hand, forearm, earlobe and the like, may be useful for sampling, and are less painful. However, they also produce lesser amounts of blood. These alternate sites therefore are generally appropriate for use only for test systems requiring relatively small amounts of fluid, or if steps are taken to facilitate the expression of the bodily fluid from the incision site.

Various methods and systems for incising the skin are known in the art. Exemplary lancing devices are shown, for example, in United States Patent Nos. Re 35,803, issued to Lange, et al. on May 19, 1998.; 4,924,879, issued to O'Brien on May 15, 1990; 5,879,311, issued to Duchon et al. on February 16, 1999; 5,857,983, issued to Douglas on January 12, 1999; 6,183,489, issued to Douglas et al. on February 6, 2001; 6,332,871, issued to Douglas et al. on December 25, 2001; and 5,964,718, issued to Duchon et al. on October 12, 1999. A representative commercial lancing device is the Accu-Chek Softclix lancet.

Patients are frequently advised to urge fluid to the incision site, such as by applying pressure to the area surrounding the incision to milk or pump the fluid from the incision. Mechanical devices are also known to facilitate the expression of bodily fluid from an incision. Such devices are shown, for example, in United States Patent Nos. 5,879,311, issued to Duchon et al. on February 16, 1999; 5,857,983, issued to Douglas on January 12, 1999; 6,183,489, issued to Douglas et al. on February 6, 2001; 5,951,492, issued to Douglas et al. on September 14, 1999; 5,951,493, issued to Douglas et al. on September 14, 1999; 5,964,718, issued to Duchon et al. on October 12, 1999; and 6,086,545, issued to Roe et al. on July 11, 2000. A representative commercial product that promotes the expression of bodily fluid from an incision is the Amira AtLast blood glucose system.

The acquisition of the produced bodily fluid, hereafter referred to as the "sampling" of the fluid, can take various forms. Once the fluid specimen comes to the skin surface at the incision, a sampling device is placed into contact with the fluid. Such devices may include, for example, systems in which a tube or test strip is either located adjacent the incision site prior to forming the incision, or is moved to the incision site shortly after the incision has been formed. A sampling tube may acquire the fluid by suction or by capillary action. Such sampling systems may include, for example, the systems shown in US Patent Nos. 6,048,352, issued to Douglas et al. on April 11, 2000, on which the first part of the independent claims is based ; 6,099,484, issued to Douglas et al. on August 8,2000; and 6,332,871, issued to Douglas et al. on December 25,2001. Examples of commercial sampling devices include the Roche Compact, Amira AtLast, Glucometer Elite and Therasense FreeStyle test strips.

The bodily fluid sample may be analyzed for a variety of properties or components, as is well known in the art. For example, such analysis may be directed to hematocrit, blood glucose, coagulation, lead, iron, etc. Testing systems include such means as optical (e.g., reflectance, absorption, fluorescence, Raman, etc.), electrochemical, and magnetic means for analyzing the sampled fluid. Examples of such test systems include those in US Patent Nos. 5,824,491, issued to Priest et al. on October 20, 1998; 5,962,215, issued to Douglas et al. on October 5, 1999; and 5,776,719, issued to Douglas et al. on July 7, 1998.

Typically, a test system takes advantage of a reaction between the bodily fluid to be tested and a reagent present in the test system. For example, an optical test strip will generally rely upon a color change, i.e., a change in the wavelength absorbed or reflected by dye formed by the reagent system used. See, e.g., US Patent Nos. 3,802,842; 4,061,468; and 4,490,465.

A common medical test is the measurement of blood glucose level. The glucose level can be determined directly by analysis of the blood, or indirectly by analysis of other fluids such as interstitial fluid. Diabetics are generally instructed to measure their blood glucose level several times a day, depending on the nature and severity of their diabetes. Based upon the observed pattern in the measured glucose levels, the patient and physician determine the appropriate level of insulin to be administered, also taking into account such issues as diet, exercise and other factors.

In testing for the presence of an analyte such as glucose in a bodily fluid, test systems are commonly used which take advantage of an oxidation/reduction reaction which occurs using an oxidase/peroxidase detection chemistry. The test reagent is exposed to a sample of the bodily fluid for a suitable period of time, and there is a color change if the analyte (glucose) is present. Typically, the intensity of this change is proportional to the concentration of analyte in the sample. The color of the reagent is then compared to a known standard which enables one to determine the amount of analyte present in the sample. This determination can be made, for example, by a visual check or by an instrument, such as a reflectance spectrophotometer at a selected wavelength, or a blood glucose meter. Electrochemical and other systems are also well known for testing bodily fluids for properties on constituents.

The present invention provides for enhancing the sampling of a bodily fluid received from an incision. It is desirable to ensure that an adequate amount of fluid is obtained to accomplished the intended testing. It is also desirable to have means to indicate that the fluid has been acquired, and that the amount is sufficient. The prior art has preferably used capillary tubes having uniform internal diameters leading to a test region. These devices work properly as long as there is a sufficient supply of fluid. However, if there is less than an amount adequate to fill the tube all the way to the test region, then the test region will not receive the fluid and no test result will be obtained. Alternatively, if there is enough fluid to reach the test region but an insufficient amount to conduct the test, then the device can yield an inaccurate test result, and the user may not be aware that the result is erroneous. The present invention provides a capillary design that reduces or eliminates these problems.

### Summary of the Invention

The present invention provides various systems and methods for the sampling of bodily fluid from an incision in the skin. The sampling is achieved through a capillary passageway having portions with different affinities for the bodily fluid. The invention encompasses separate sampling devices as well as combination systems including incising, expression and/or testing systems.

In accordance with one aspect of the present invention, there is provided a sampling device for acquiring a sample of a bodily fluid which includes a body defining a capillary passageway and an inlet opening, the capillary passageway including a first portion communicating with the inlet opening and a second portion communicating with the first portion and located downstream of the first portion. The first portion has a first capillary affinity for the bodily fluid, and the second portion has a second capillary affinity for the bodily fluid which is greater than the first capillary affinity, wherein the capillary affinities may differ based upon various factors, including surface treatment and coating, wicking material or material selection. The capillary passageway may also include three or more portions having different capillary affinities for the bodily fluid. The capillary may include fluid detectors to identify the progress of fluid within the passageway.

The present invention further encompasses the combination of the foregoing sampling system with incising, expressing and/or testing systems, particularly in a single, integrated device. The present invention also contemplates the associated methods for sampling bodily fluid from an incision, including the combination with methods for incising, expressing and/or testing of the bodily fluid.

### Brief Description of the Drawings

FIG. 1 is a side, elevational view of an integrated fluid testing device utilizing a capillary sampling system according to one embodiment of the present invention.

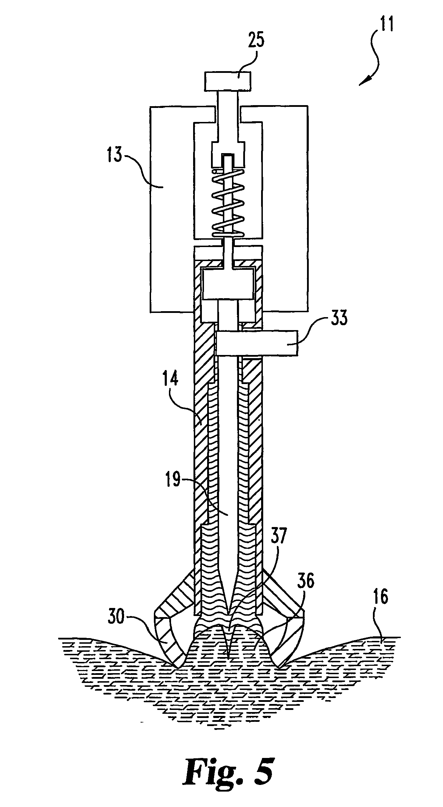

FIGS. 2-5 are partial, cross-sectional views of the fluid testing device of FIG. 1, showing in particular the acquisition of the fluid by the capillary sampling system.
FIG. 6 is a side, cross-sectional view of a test strip including a capillary sampling system in accordance with an alternate embodiment of the present invention.
FIG. 7 is a top, plan view of the test strip of FIG. 6, partially in cross section.
FIG. 8 is a side, cross-sectional view of the test strip of FIG. 6, showing the expression system in the constricting position.
FIG. 9 is a side, cross-sectional view of the test strip of FIG. 6, showing the bodily fluid being acquired by the capillary passageway in the test strip.
FIG. 10 is a side, cross-sectional view of a lancing device useful in combination with a capillary sampling system of the present invention.
FIG. 11 is a side, elevational view of a lancet holder useful in the device of FIG. 10.
FIG. 12 is a partial, cross-sectional view of the skin-engaging portion of the device of FIG. 10, and further showing a test strip mounted therein.
FIG. 13 is a cross-sectional view of the device of FIG. 12 taken along the line 13-13 and viewed in the direction of the arrows.

### Description of the Preferred Embodiment

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated devices and methods, and such further applications of the principles of the invention as illustrated therein being contemplated as would normally occur to one skilled in the art to which the invention relates.

The present invention provides a variety of devices and methods which separately or in combination are useful in enhancing the sampling of fluid from an incision in the skin. This sampling of the fluid utilizes a passageway sized to receive the fluid and transport it by capillary action. The invention particularly relates to the use of a capillary passageway with separate portions having different capillary affinities for the fluid. In this way, fluid is fully transported through the device, provided that there is sufficient fluid to fill at least the first portion of the passageway. Once the fluid contacts the subsequent capillary portion(s), the greater affinity of the subsequent portion for the fluid continues to move the fluid along the device.

The term "capillary affinity" is used to identify the strength of the attraction between the fluid and the subject portion of the device to cause the fluid to move into and through that portion of the device. If the fluid is in contact with two portions having different capillary affinity, the fluid will move to the portion having the higher affinity. For example, in general a passageway having a smaller cross-sectional area has a stronger attraction for a fluid than a similar passageway having a larger cross section. In that instance, if a passageway includes adjacent portions having different cross-sectional areas, then fluid filling the larger passageway and contacting the smaller passageway will move into the smaller passageway due to the greater capillary affinity of the smaller passageway. This difference in capillary affinity for adjacent portions is also referred to herein as a stepped capillary, in that there is a distinct step or difference in the capillary affinity of the two portions.

As is well known in the art, capillary affinity of portions of a passageway may be varied in several ways. As indicated, passageways having different cross-sectional areas but otherwise being identical will generally have different capillary affinities for a given fluid, usually with the smaller cross section passageway having the higher affinity. The provision of a suitable material within a passageway can also increase the capillary affinity associated with that portion of the passageway. Different materials may have different affinities for a fluid, such that forming the passageway from different materials will provide a change in the capillary affinity between those portions. Capillary affinity can also be changed by treating or coating the capillary surface, for example to provide a resulting surface that is more or less hydrophilic. The present invention is operable in respect to those ways in which the capillary affinity is varied that are defined in the independent claims. For purposes of illustration, a capillary passageway having changes in cross-sectional area as the means to vary the capillary affinity along the length of the passageway.

The fluid is obtained from an incision formed in the surface of the skin. The incising of the skin may be accomplished by any suitable means, including cutting with a mechanical instrument, laser, high speed fluid stream, etc. Of these, lancing the skin is most common and is preferred, and specific descriptions herein use lancing for purposes of example. It will be appreciated, however, that lancing is only exemplary, and all forms of making an incision in the skin are included. As used herein, the term "incision" is intended to cover any opening in the skin that permits direct access to the bodily fluid. The term "incising" is intended to mean generally any way to form an incision in the skin to enable fluid to be accessed directly. The term "incision site" is intended to include the site where an incision either has been or will be formed, unless from the context or express language it is clear otherwise.

The depth of penetration generally controls the fluid produced, particularly in combination with the characteristics of the incision site. The present invention is useful with various bodily fluids, including blood or interstitial fluid. The incising device may be configured for production of either blood or interstitial fluid, for example, by controlling the distance which the incising device extends into the user's skin. For example, a depth of 0.25 mm to 4 mm will typically produce blood from the dermis, while a depth of 0.05 mm to 0.5 mm will produce interstitial fluid from the epidermis.

It will be appreciated from the following description that the present invention is useful independently of the presence or type of incising, expressing or testing systems. In certain embodiments, the present invention may comprise devices, and associated methods, which are limited only to sampling of fluid from an incision. In other embodiments, the sampling mechanisms and methods are combined with incising, expressing and/or testing systems. The present invention finds particular advantage in combination with such other systems as a part of an overall integrated device.

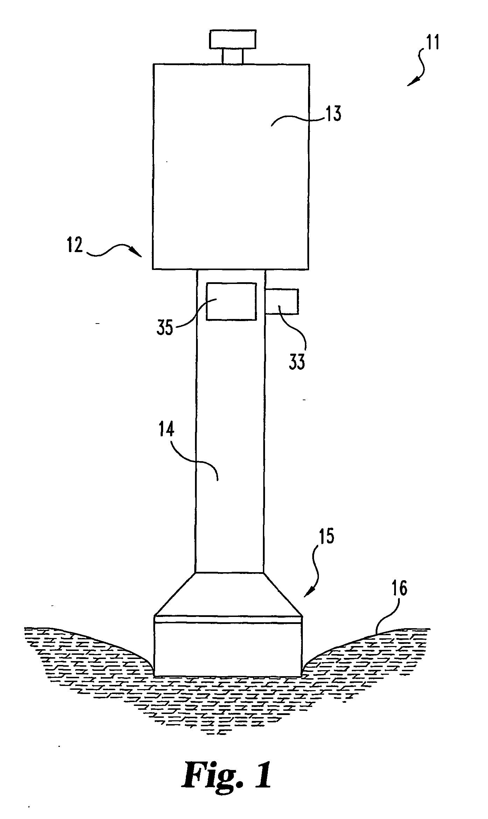

Referring to the drawings, an integrated fluid monitoring device is shown in FIG. 1. The integrated device 11 includes a housing 12 which includes or supports components operable to lance, express, sample and test bodily fluids. The housing includes a first member 13, a cylindrical extension member 14, and an expression system 15. The device 11 is shown in FIG. 1 as being contacted against the skin 16 in the position for expression of bodily fluid.

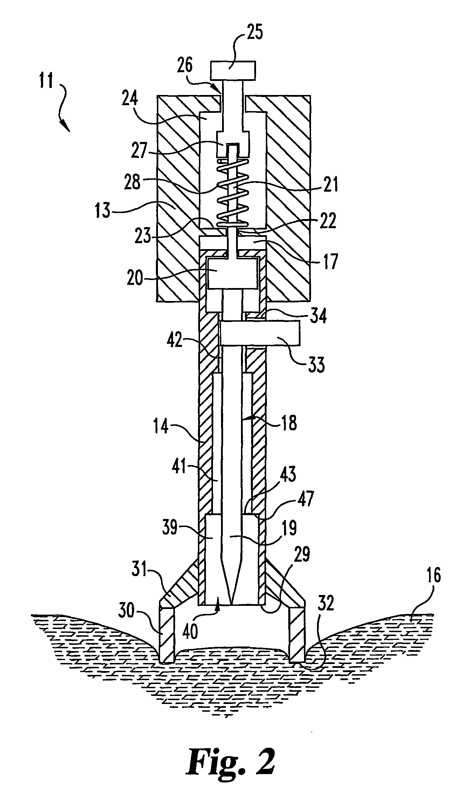

The components of the integrated device 11 are shown in detail beginning in FIG. 2. The cylindrical member 14 is mounted within a cavity 17 defined by the first member 13, and is secured therein, such as by a press fit or by gluing. The cylindrical member 14 defines an interior passageway 18, and a lancet 19 is received therein. The space between the lancet and the cylindrical member therefore defines an annular passageway, which is sized to provide a capillary attraction to the desired bodily fluid, as later described.

The lancet 19 is mounted to a lancet carrier 20 which includes an extension 21. The extension passes through an aperture 22 formed in an interior wall 23 of the housing 12. The housing 12 further defines a chamber 24 in which the extension 21 is received. A lancet button 25 is received through an aperture 26 in the member 12 and includes a mounting yoke 27 which is connected with the lancet carrier extension 21. A coil spring 28 is positioned around the extension 21 and is bears at one end on the yoke 27, and at the other end on the wall 23. In this manner, pressure applied against the button 25 will urge the lancet beyond the distal end 29 of the cylindrical member 14 for lancing the skin. Upon release of the downward pressure, the spring 28 will withdraw the lancet back into the cylindrical member 14, thereby removing the lancet from the incision formed in the skin.

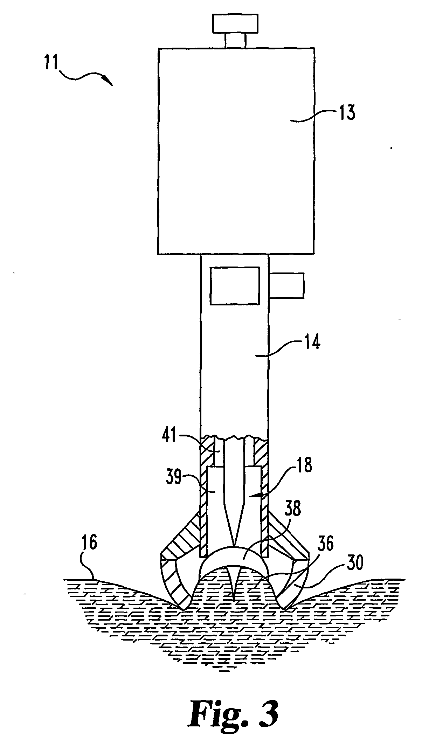

The device 11 further includes an expression system 15 attached to the cylindrical member 14. In particular, the expression system includes a cylindrical expressing member 30 secured to a support 31 which is in turn attached to or formed integrally with the cylindrical member 14. The expressing member 30 is deformable to facilitate the expression of fluid from an incision positioned interior of the member. The expressing member has an initial condition in which the skin-engaging surface 32 contacts the skin at a radially-outward position (FIG. 2). Upon further pressing the device 11 against the skin, the member 30 deforms inwardly, thereby grasping and moving the skin upward and inward to a constricted position (FIG. 3). This movement applies pressure against the skin to hold bodily fluid within the constricted area and to urge the fluid toward the center.

A test strip 33 is received through an aperture 34 in the wall of the cylindrical member 14. The test strip extends within the annular passageway between the lancet 19 and the interior of the cylindrical member 14, and therefore is in position to be contacted by fluid received in the passageway. A window is located in the side of the cylindrical member 14 at a position to allow the test strip to be viewed from the exterior of the device. Therefore, the results of a reaction between the bodily fluid and the test strip can be observed through the window 35. Alternative test systems, including optical and electrochemical systems for example, are equally useful in accordance with the present invention.

The integrated device is operable to provide complete lancing, expressing, sampling and testing of a bodily fluid as follows. As shown in the drawings, the device 11 is initially positioned against the skin at the location desired for fluid acquisition. The device is then pressed against the skin sufficiently to deform the expressing member 30, as shown in FIG. 3. This results in the creation of a raised pinch of skin 36. A force is then applied to the button 25 to move the lancet downwardly into the skin to form an incision . The force is immediately released from the button and the lancet retracts from the incision into the cylindrical member, as shown in FIG. 3. A droplet of bodily fluid 38 will begin to form at the incision site, facilitated by the expressive forces applied to the skin by the expressing member 30.

As the droplet grows in size, it contacts the inlet opening 40 of the passageway 18 and is drawn in by capillary action. The fluid sample continues to be drawn into the passageway until it contacts the test strip 33. The test strip is selected to provide a test of the desired constituent or property of the bodily fluid being sampled. The results are obtained by optical detection of the reaction through the window 35.

In accordance with the present invention, the passageway 18 includes portions having different capillary affinities for the bodily fluid. For example, the passageway includes a first portion 39 communicating with the inlet opening 40. The passageway further includes a second portion 41 communicating with the first portion 39 and located on the side of the first portion opposite the inlet opening. The second portion is thereby positioned downstream of the first portion as the bodily fluid moves through the device. The passageway also includes a third portion 42 downstream of the second portion 41.

The capillary affinity of each portion of the passageway is provided to be greater than the affinity of the preceding, upstream portion of the passageway. In the example as shown in the drawings, and not according to the invention, the greater capillary affinity is obtained by the fact that the cross-sectional area of each of the successive portions 41 and 42 is reduced from that of the previous, upstream portion. In addition, the device 11 includes test strip 33 which also acquires the bodily fluid by virtue of the capillary affinity of the strip material. The strip material is selected to have a capillary affinity which is greater than that of the preceding passageway portions. Therefore, when the fluid passes up through the passageway and contacts the test strip, it is absorbed by the test strip and is therefore available for testing.

The operation of the device 11 functions more particularly as follows. As previously described, the device is pressed against the skin and the lancet 19 is advanced to incise the skin. As the drop of bodily fluid is formed, it will grow to a size that it contacts the inlet opening 40 of the passageway 18 (FIG. 3). The fluid will then be drawn into the passageway by capillary action, and will continue to move up the first portion 39 of the passageway to the extent that there is sufficient fluid to do so.

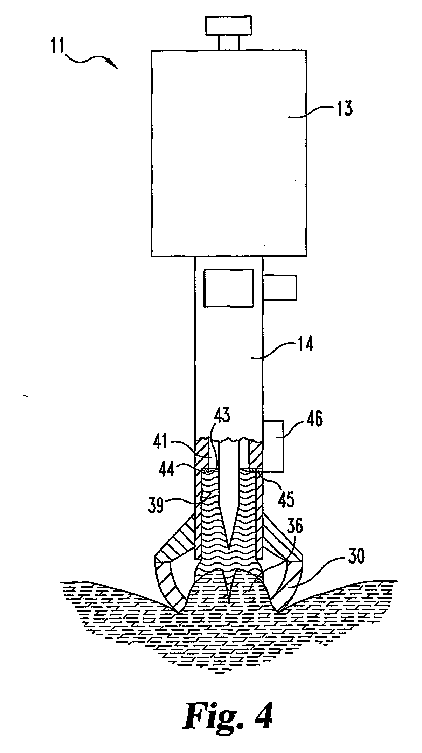

Provided there is enough fluid, the fluid will move up the first portion of the passageway until it has contacted the second portion 41 (FIG. 4). The greater capillary affinity of the second portion will then draw the fluid into and through the second portion to the extent that there is sufficient fluid. Preferably, the volume of the second portion of the passageway is the same as or smaller than the volume of the first portion of the passageway. Consequently, when there is sufficient fluid to fill the first portion there will also be a sufficient amount to fill the second portion. Similarly, the fluid will then move up to the point that it contacts the third portion 42 of the passageway and will be drawn into the third portion. The volume of the third portion is also preferably selected to be the same as or smaller than the volume of the second portion in order to assure that filling of the second portion will provide a sufficient amount to also fill the third portion. Finally, the test strip is sized to provide that the amount of fluid required to fill the third portion will also be sufficient to wet the test strip to an extent sufficient to be accurately tested by the strip.

It will be appreciated that the invention therefore provides a sampling device that affords distinct advantages. For example, the sizing of the various passageway portions can be selected to assure that there is sufficient sample to obtain accurate test results. It will also be apparent that the inventive devices may include any number of passageway portions, provided that subsequent, downstream portions have capillary affinity greater than the immediately adjacent, upstream portions.

The changes in capillary affinity have been depicted with respect to variations in cross-sectional area. However, various other techniques known for providing desired capillary affinities may be used. For example, a wicking material could be disposed within one of the capillary portions such that the affinity of the wicking material is greater than the affinity of the upstream portion. Successive wicking materials of different capillary affinities could be used within a passageway of uniform cross-sectional area in order to provide the successive portions as described herein. The interior surfaces defining the capillary passageway could be treated to have greater or lesser capillary affinity, such as by varying the hydrophilicity of the portions. Alternatively, the passageway portions could be defined by different materials, including coatings, to provide the different capillary affinities. In summary, a particular passageway will have a given capillary affinity for the subject bodily fluid - whether due to surface treatment, coating, material selection or wicking material. The present invention contemplates any combination of passageway portions that have the required increases in capillary affinity in the direction of desired flow of the bodily fluid within the device.

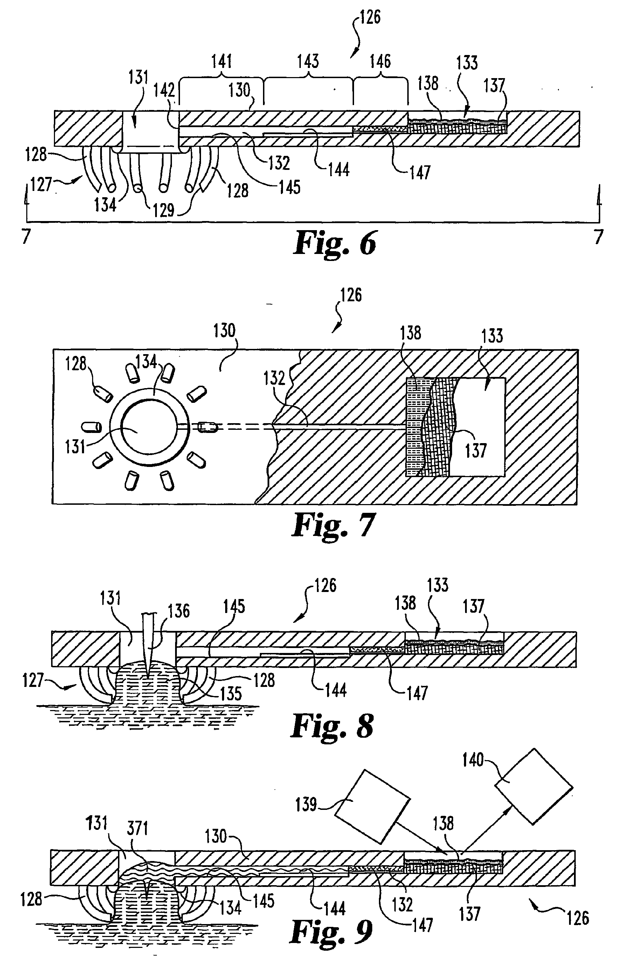

Referring in particular to FIGS. 6-9, there is shown a second embodiment of the present invention in combination with lancing, expressing and testing systems. The device comprises a test strip 126 including a constricting system 127 attached to the underside thereof. The constricting system includes several discrete, deformable elements 128, each element defining a surface 129 to engage the skin and move it inwardly to constrict the skin. The elements deform in a manner to move the skin-engaging surfaces in a radially-inward direction from the first positions to the second positions, and the elements are therefore preferably positioned to be diametrically-opposed, or equi-radially spaced about the incision site.

The strip 127 further includes components for the purposes of incising the skin and collecting the produced fluid sample. The test strip includes a body 130 defining an opening 131, a capillary passageway 132, and a test area 133. A sealing ring 134 is attached to or formed integrally with the underside of the body 130 in a position surrounding the opening 131 and interior of the deformable arms 128.

The capillary passageway 132 is constructed in accordance with the present invention and includes three successive portions. The first portion 141 communicates with the inlet opening 142. The second portion 143 is defined by a region which has been coated with a material 144 having greater hydrophilicity than the surface 145 in the first portion 141. The third portion 146 is defined by the presence of a wicking material 147 extending throughout that portion of the passageway. As previously described, the capillary affinities of the successive portions are selected to provider greater drawing force for the bodily fluid in the intended direction of travel for the fluid.

The use of the test strip system 126 proceeds as follows. The test strip 126 is pressed against the skin such that the arms 128 engage the skin and deform inwardly, thereby creating and retaining a bulged skin area 135. The skin is drawn upward and inward to an extent that it bears against the sealing ring 134, forming a fluid tight seal therewith. This assures that any fluid exiting the incision will be retained within the opening 131, rather than moving out under the test strip body. The sealing ring further functions to press against the skin, thereby providing an additional expression force, and pulling on the skin to open the incision when formed. Also, the contact of the skin with the sealing ring locates the skin at a controlled position to facilitate the formation of the incision at a desired depth and position.

A lancing device 136 is extended downwardly through opening 131 to lance the skin to the desired, controlled depth. The lancet is then withdrawn (FIG. 9) and bodily fluid 371 is allowed to form at the incision site. When the fluid accumulates to a sufficient extent, it contacts the entrance of the passageway 132 and is drawn into and through the passageway by capillary action. The fluid moves through the successive portions 141, 143 and 146, respectively, of the passageway 132. The fluid is subsequently drawn into the test area 133, such as by wicking into an absorbent material 137, and there contacts the test reagent 138 positioned on top of the wicking material.

The fluid is thereby presented in the test area and can be tested by conventional means, such as by reacting the fluid with the test reagent and analyzing the reaction product by optical or electrochemical means. For example, shown diagrammatically in FIG. 9 is a light source 139 for directing light against the test reagent, and a blood glucose meter 140 for receiving light reflected from the test reagent. In conventional fashion, the meter analyzes the reflected light to determine the result of the reaction between the bodily fluid and the test reagent. In this manner, a wide variety of analytes and properties of the fluid may be determined. Such test systems are well known in the art and therefore are not further described herein.

This embodiment provides another example of an integrated device which combines the sampling system with incising, expressing and/or testing of the bodily fluid. A test strip of this type is preferably used once and then discarded. In use, the test strip is pressed against the skin to automatically deform the expression arms, thereby grasping and constricting the skin. Bodily fluid expressed from the incision is collected and tested, and the test strip is thereafter disposable.

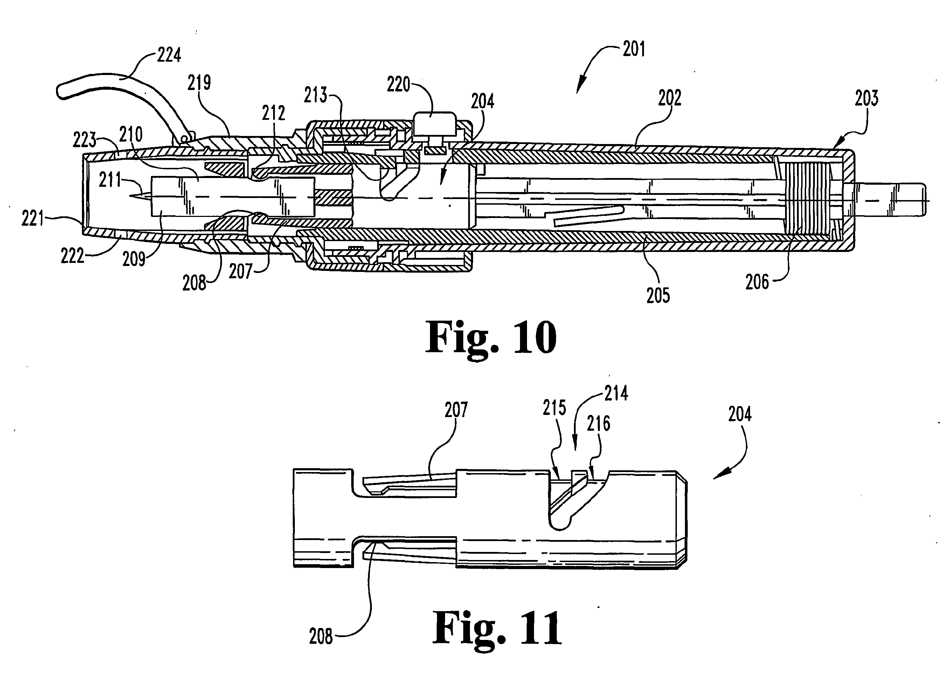

As a further example of the present invention, shown in FIGS. 10-13 is a typical lancing device 201, except that it has been modified to include a capillary sampling system in accordance with the present invention. The basic lancing device is further described in United States Patent No. Re 35,803 Therefore, for illustrative purposes, only the major components of said device are shown in the drawings and described herein.

The lancing device 201 includes a housing 202 which contains a lancet drive mechanism 203 and a lancet holder 204. The drive mechanism includes a rotatable sleeve 205 and a spirally-wound, coiled spring 206 coupled between the housing and the rotatable sleeve. The lancet holder 204 is longitudinally slidable within the sleeve 205 and includes arms 207 with end lugs 208 that are receivable within recesses formed in a lancet component. The lancet component 209 includes a body 210 and a lancet tip 211. The lancet body defines a circumferential recess 212 which receives the end lugs 208 of the arms of the lancet holder 204. The lancet 209 is thereby longitudinally movable inside of the sleeve 205 in concert with the movement of the lancet holder 204.

The rotatable sleeve 205 includes a drive pin 213, and the lancet holder 204 defines a driver cam 214. The driver cam includes a first cam segment 215 to allow for cocking of the mechanism. The driver cam further includes a second, symmetrical, arcuate cam segment 216 to provide for projection and withdrawal of the lancet tip relative to the housing opening 217 formed in the pressing member 218 of the housing. An outer ring 219 connects with the rotatable sleeve 205 and upon rotation of the outer ring the sleeve is also rotated to tension the spring 206 as the drive pin 213 moves within the first cam segment 215. The rotatable sleeve automatically locks once in the fully tensioned position.

Upon pressing a lock release button 220, the sleeve rotates back to its original position. During this return rotation, the drive pin 213 moves within the second cam segment 216, causing the lancet holder and lancet initially to translate longitudinally of the sleeve 205 and housing 202 in a direction to drive the lancet tip to incise the skin. The lancet tip 211 is immediately thereafter withdrawn by operation of the second cam segment 216 of the lancet holder.

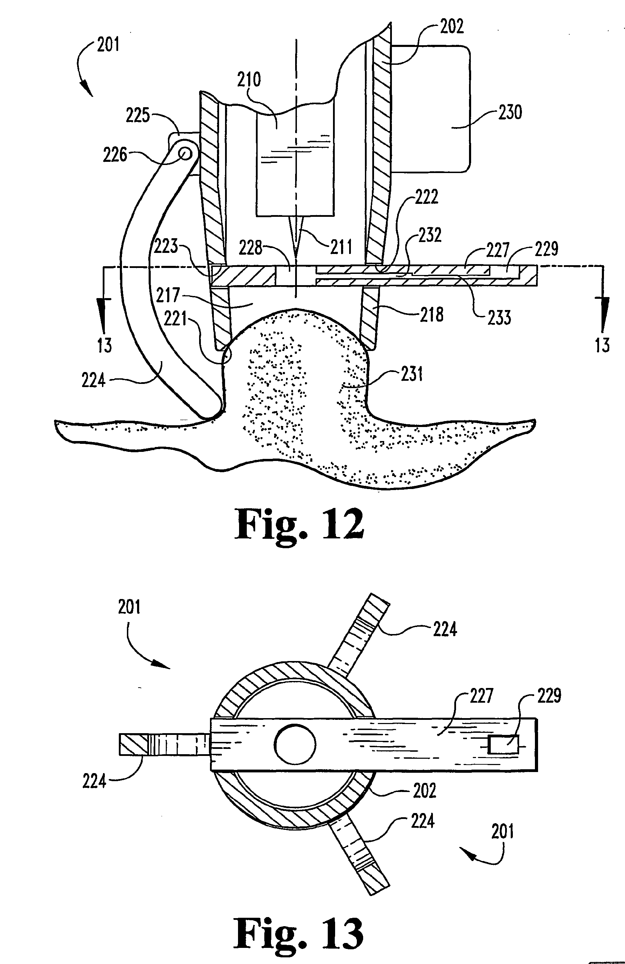

The pressing member extends to an annular surface 221 and defines slots 222 and 223 adjacent thereto. Three pivoting expression arms 224 are secured to the housing in equi-radially spaced positions by means of yokes 225 and pins 226. Each arm 224 has a first, spread position (FIG. 10), and is movable from this initial position to the second, constricting position (FIG. 12).

A test strip 227 is received within the slots 222-223 and includes an aperture 228 which is thereby positioned in line with the lancet 211. The test strip includes a capillary passageway that extends from an inlet opening which communicates with the aperture 228 to a test region 229. The capillary passageway includes a first portion 232 and a second portion 233 having a higher capillary affinity than the first portion 232. The test region includes suitable reagent to interact with the bodily fluid which is received in the test region. An optical test device 230 is mounted to the housing and is positioned to evaluate the results of the reaction in the test region.

In accordance with the present invention, the integrated device 201 is operable as follows. The device is pressed against the skin whereupon the arms 224 are manipulated from the open position to the constricting position (FIG. 12). The skin 231 is thereby drawn in to form a raised pinch of skin that bears against the annular surface 221. The lancet 211 is then advanced through the aperture 228 in the test strip and incises the skin. As a fluid droplet forms, it contacts the capillary passageway of the test strip 227 and is transported to the test region 229. The fluid then reacts with the reagent provided in the test region, and the results are read by the test device 230.

More particularly, the fluid will enter and move along the first portion 232 as the fluid is available from the incision. At some point in time, the fluid will contact the second portion 233. When that occurs, the greater affinity of the second portion will draw the fluid from the first portion. The fluid thereby moves along the passageway sufficiently to contact the wicking material (not shown) in the test region 229.

The present invention provides the opportunity to predetermine the volume required to move to the test area. For example, in order for the fluid to move from one capillary portion to the next, it is necessary that there be a sufficient volume of fluid to fill the upstream portion (having the lower capillary affinity). The fluid will not move into the subsequent passageway portion unless it contacts at least the beginning of that portion, and that requires that the fluid first fill the passageway portion that it is in. In this context, the volume of a portion of the passageway is the volume available for the fluid to fill. For example, for an open passageway the volume would be the entire volume of the portion, whereas for a passageway including a wicking material, the volume would be the void volume available within that portion.

It is an aspect of the present invention that the volume of one or more of the passageway portions is predetermined in order to serve a given function. For example, in a preferred embodiment, the volume of one of the passageway portions, more preferably the first portion, is sized to be at least large enough to supply an amount of fluid to the test region required for the test to be conducted. In another preferred embodiment, each successive, downstream portion of the passageway is sized to have a volume that is the same as, or more preferably less than, the immediately preceding, upstream portion of the passageway.

In an alternative embodiment, the passageway may be configured to have a second portion having a larger volume than a preceding first portion. The second portion may then function as an intermediate collection area to provide for the accumulation of a volume of fluid desired for subsequent testing. This larger second portion would serve the same function, as previously described, to require that a preselected volume be acquired before the fluid is moved on to the test region.

In a related aspect of the present invention, there is a further advantage in the fact that a larger, preceding passageway portion must be filled before the fluid continues through the device. For example, in prior art embodiments it has been possible for there to be an amount of fluid which would wet the test region, but which would be an insufficient amount to provide an accurate test result. It was then difficult for the user to know whether the same device should be recontacted with the source of bodily fluid in order to obtain a larger sample. In contrast, the present invention permits the user to acquire an amount of bodily fluid, and if the device does not perform the test because the amount is insufficient, then the user may re-contact the fluid source to obtain additional bodily fluid until the fluid moves to the test region.

The present invention further is particularly useful in combination with a fluid detector operable to indicate the presence of fluid within the capillary passageway. Fluid detectors are known in the art for detecting when a sampled bodily fluid has reached a given point along the path of a sampling and/or testing device. However, the present invention provides additional advantage over prior art devices due to the presence of the separate passageway portions. For example, a fluid detector positioned in the second portion of the capillary passageway will provide an indication that the first portion has filled. Thus, for the preferred embodiments in which the first portion is sized to assure sufficient fluid for the downstream testing, the detection of fluid in the second portion also is an indication that the device has acquired enough fluid for a successful test to be conducted. This fact can then be readily displayed to the user by a visible or audible signal and the device can be withdrawn from the skin site.

Referring to FIG. 4, for example, the device 11 includes a passageway 18 having first and second portions 39 and 41, respectively, meeting at a juncture 43. A fluid detector 44 is located at the juncture and connects through lead 45 to a detection device 46. In the embodiment shown in the drawings, the two passageway portions are defined by different cross-sectional areas. This defines a shoulder on which the fluid detector may be placed. However, it will be appreciated that the juncture between adjacent passageway portions may be defined by other structures, and the fluid detector may be placed on or adjacent to whatever form of juncture exists between the adjacent portions.

The device 46 functions to operate the detector 44 and receive a signal or other indication when fluid has contacted the fluid detector 44. The device 46 is then also functional to provide a signal to the user to confirm that fluid has been detected. The device 46 may also be operably connected with the test system of the overall device 11 to signal to the test system that sufficient fluid has been acquired to conduct the test. In certain embodiments, the detection device may trigger the starting of the test system, preferably with a brief time delay calculated to allow the fluid sample to continue through the remaining portions of the passageway 18 to the test strip 33 (or other testing device).

The detector 44 and associated components may comprise any of a variety of well known detection systems. The detector may be as simple as a color indicator, visible from outside the device 11, which changes color upon contact with the bodily fluid. The user then observes the color indicator for the requisite color change in order to know that fluid has been detected. Another example of a fluid detector is a pair of closely-spaced electrodes to which a voltage is applied. When fluid reaches the electrodes and bridges the gap between the electrodes, the circuit is completed and current flowing through the electrodes is used to signal the fluid detection. These and other fluid detectors are well known in the relevant art, and further descriptions are therefore not provided. However, it will be understood that such fluid detectors operate substantially independently of the present invention and are readily adapted for use herein, and that the use of all such detectors is contemplated herein.

The location of the fluid detector also is not critical to the present invention. In a preferred embodiment, the fluid detector is located at the juncture between the first and second portions. However, the fluid detector may also be usefully located at other points. The detector may be located, for example, at the juncture of any two adjacent passageway portions, or at the juncture between the final passageway portion and the test region. The detector alternatively may be located within any of the passageway portions, or in the test region. Also, a plurality of fluid detectors may be used to detect the presence of fluid in different locations, or to monitor the movement of fluid through the passageway and test region.

It will be appreciated from the foregoing descriptions that the present invention is useful independently of the presence or type of incising, expressing or testing systems. In certain embodiments, however, the sampling devices and methods are combined with incising, expressing, and/or testing systems, particularly in a single, integrated device. Because the sampling is achieved essentially independently of these other systems, the sampling system is readily adapted as an additional component of such devices. It will similarly be appreciated that the integrated device may also combine testing means, as demonstrated herein, to test desired constituents or characteristics of the fluid sample that has been acquired. The invention will therefore be seen to be useful in combination with a wide range of incising, expressing and testing systems, including those herein described in the description of the prior art and elsewhere, and the disclosures of such patents are hereby incorporated by reference.

Such an integrated device preferably operates such that the device does not have to be repositioned at any time during the process of incising, expressing, and/or sampling. More specifically, the device preferably carries incising, expressing, sampling and testing systems to perform a complete, integrated monitoring of the bodily fluid. In accordance with this approach, the device is moved against the skin and is maintained in this position while the incision is formed, and also while the resulting fluid droplet develops and is carried into the sampling device. The fluid is then analyzed by the test system and the result of the analysis is provided to the user. All of these actions therefore may be accomplished by a single, integrated unit, providing a simple, quick and reliable method for acquiring and testing a bodily fluid.

Moreover, the combination of the various systems in a single unit assures that the separate systems will be properly coordinated in use. The timing for the formation of the constricted pinch of skin and the lancing of the skin can be controlled automatically, or by manual operation by the user. The positions of the incision site and of the sampling capillary tube are predetermined to optimize the acquisition of the fluid formed at the incision site. This reduces the potential for a user to fail to successfully collect the fluid that is produced.

While the invention has been illustrated and described in detail in the drawings and foregoing description, the same is to be considered as illustrative and not restrictive in character, it being understood that only the preferred embodiment has been shown and described and that all changes and modifications that come within the scope of the invention are desired to be protected.

## Claims

1. A sampling device (11) for acquiring a sample of a bodily fluid which comprises:
a body (14) defining a capillary passageway (18) and an inlet opening (40) the capillary passageway including a first portion (39) communicating with the inlet opening and a second portion (41) communicating with the first portion and located on the side of the first portion opposite the inlet opening; the first portion of the capillary passageway having a first capillary affinity for the bodily fluid; the second portion of the capillary passageway having a second capillary affinity for the bodily fluid which is greater than the first capillary affinity, **characterized in that** the greater capillary affinity is due to surface treatment, coating, material selection or wicking material.

2. The device of claim 1 in which the second portion of the capillary passageway contains a wicking material; the capillary affinity of the wicking material being greater than the capillary affinity of the first portion of the capillary passageway.

3. The device of claim 1 in which the interior surface first portion is coated with a material which is different than the interior surface of the second portion,

4. The device of claim 1 and which further includes an expression member positioned to express the bodily fluid for reception by the inlet opening of the capillary passageway.

5. The device of claim 1 and which further includes a test region communicating with the second portion of the capillary passageway, the test region having a capillary affinity for the bodily fluid which is greater than the capillary affinity of the second portion.

6. The device of claim 1 and which further includes a test means for testing the bodily fluid in the second portion of the capillary passageway.

7. The device of claim 6 and which further includes a test region communicating with the second portion of the capillary passageway, the test region having a capillary affinity for the bodily fluid which is greater than the capillary affinity of the second portion, said test means being for testing the bodily fluid in the test region.

8. The device of claim 1 and which further includes an incising mechanism positioned to incise a person's skin to produce the bodily fluid to be received by the inlet opening of the capillary passageway.

9. The device of claim 8 in which said incising mechanism comprises a lancing device positioned to lance the person's skin at a location adjacent to the inlet opening of the capillary passageway.

10. The device of claim 9 in which said lancing device comprises an annular capillary lancet, the annular capillary lancet including a capillary tube and a lancet received within the capillary tube, said body including the capillary tube and the capillary tube defining the capillary passageway.

11. The device of claim 9 in which said body comprises a test strip.

12. The device of claim 8 and which further includes an expression member positioned to express the bodily fluid for reception by the inlet opening of the capillary passageway.

13. The device of claim 8 and which further includes a test region communicating with the second portion of the capillary passageway, the test region having a capillary affinity for the bodily fluid which is greater than the capillary affinity of the second portion.

14. The device of claim 13 and which further includes an expression member positioned to express the bodily fluid for reception by the inlet opening of the capillary passageway.

15. The device of claim 1 in which the capillary passageway includes at least a third portion communicating with the second portion and located on the side of the second portion opposite the first portion, the capillary passageway thereby including a plurality of successive capillary portions, each of the successive portions of the capillary passageway having a greater capillary affinity than the preceding portion moving in the direction away from the inlet opening.

16. The device of claim 15 and which includes a test region communicating with the final one of the successive portions of the capillary passageway, the test region having a capillary affinity for the bodily fluid which is greater than the capillary affinity of the final one of the successive portions.

17. The device of claim 16 and which further includes a test means for testing bodily fluid in the test region.

18. The device of claim 1. in which the first portion of the capillary passageway has a predetermined volume.

19. The device of claim 18 in which the predetermined volume is a volume sufficient to fill a subsequent portion of the capillary passageway.

20. The device of claim 181 in which the predetermined volume is a volume sufficient for conducting a test of the sampled bodily fluid.

21. The device of claim 18 and which includes a test region communicating with the second portion of the capillary passageway, the test region having a capillary affinity for the bodily fluid which is greater than the capillary affinity of the second portion.

22. The device of claim 21 in which the predetermined volume is a volume sufficient to fill the second portion of the capillary passageway.

23. The device of claim 1 and which further includes a fluid detector positioned to detect when the sampled bodily fluid has been received at a location in the capillary passageway on the side of the first portion of the capillary passageway opposite the inlet opening.

24. The device of claim 23 in which said fluid detector is positioned to detect when the sampled bodily fluid has been received within the second portion of the capillary passageway.

25. The device of claim 1 and which further includes a fluid detector positioned to detect when the first portion of the capillary passageway has been filled.

26. The device of claim 25 in which the second portion of the capillary passageway is adjacent to and has a juncture with the first portion of the capillary passageway, said fluid detector being positioned to detect when the sampled bodily fluid has reached the juncture between the fist portion and the second portion.

27. The device of claim 1 in which said body comprises a single, unitary component.

28. The device of claim 1 in which the volume of the second portion of the capillary passageway is greater than the volume of the first portion of the capillary passageway.

29. The device of claim 1 in which the capillary passageway includes at least a third portion communicating with the second portion and located on the side of the second portion opposite the first portion, the capillary passageway thereby including a plurality of successive capillary portions, each of the successive portions of the capillary passageway having a greater capillary affinity than the preceding portion moving in the direction away from the inlet opening, at least one of the successive capillary portions having a volume greater than the volume of at least one of the preceding capillary portions.

30. A method for handling a sample of a bodily fluid which comprises:
providing a sampling device comprising a body defining a capillary passageway and an inlet opening, the capillary passageway including a first portion communicating with the inlet opening and a second portion adjacent to and communicating with the first portion and located on the side of the first portion opposite the inlet opening; the first portion of the capillary passageway having a first capillary affinity for the bodily fluid; the second portion of the capillary passageway having a second capillary affinity for the bodily fluid which is greater than the first capillary affinity; contacting the inlet opening of the sampling device with a bodily fluid, said contacting comprising providing a quantity of the bodily fluid sufficient to completely fill the first portion of the capillary passageway and to at least contact the second portion of the capillary passageway, whereby the greater affinity of the second portion causes the bodily fluid to be drawn into the second portion from the first portion, **characterized in that** the greater capillary affinity is due to surface treatment, coating, material selection or wicking material.

31. The method of claim 30 and which, after said contacting, further includes testing the bodily fluid.

## Patentansprüche

1. Entnahmeeinrichtung (11) zum Erfassen einer Probe einer Körperflüssigkeit, die Folgendes umfasst:
einen Körper (14), der einen Kapillardurchgang (18) und eine Einlassöffnung (40) definiert, wobei der Kapillardurchgang einen ersten Abschnitt (39), der mit der Einlassöffnung in Verbindung steht, und einen zweiten Abschnitt (41) aufweist, der mit dem ersten Abschnitt in Verbindung steht und an der der Einlassöffnung gegenüberliegenden Seite des ersten Abschnitts angeordnet ist, wobei der erste Abschnitt des Kapillardurchgangs eine erste Kapillaraffinität für die Körperflüssigkeit hat und der zweite Abschnitt des Kapillardurchgangs eine zweite Kapillaraffinität für die Körperflüssigkeit hat, die größer als die erste Kapillaraffinität ist, **dadurch gekennzeichnet, dass** die größere Kapillaraffinität auf Oberflächenbehandlung, Beschichtung, Materialauswahl oder dochtartiges Material zurückzuführen ist.

2. Einrichtung nach Anspruch 1, wobei der zweite Abschnitt des Kapillardurchgangs ein dochtartiges Material enthält, wobei die Kapillaraffinität des dochtartigen Materials größer als die Kapillaraffinität des ersten Abschnitts des Kapillardurchgangs ist.

3. Einrichtung nach Anspruch 1, wobei die innere Fläche des ersten Abschnitts mit einem Material beschichtet ist, das sich von der inneren Fläche des zweiten Abschnitts unterscheidet.

4. Einrichtung nach Anspruch 1, ferner mit einem Ausdrückelement, das so positioniert ist, dass es die Körperflüssigkeit zur Aufnahme durch die Einlassöffnung des Kapillardurchgangs ausdrückt.

5. Einrichtung nach Anspruch 1, ferner mit einem Testbereich, der mit dem zweiten Abschnitt des Kapillardurchgangs in Verbindung steht, wobei der Testbereich eine Kapillaraffinität für die Körperflüssigkeit hat, die größer als die Kapillaraffinität des zweiten Abschnitts ist.

6. Einrichtung nach Anspruch 1, ferner mit einem Testmittel zum Testen der Körperflüssigkeit im zweiten Abschnitt des Kapillardurchgangs.

7. Einrichtung nach Anspruch 6, ferner mit einem Testbereich, der mit dem zweiten Abschnitt des Kapillardurchgangs in Verbindung steht, wobei der Testbereich eine Kapillaraffinität für die Körperflüssigkeit hat, die größer als die Kapillaraffinität des zweiten Abschnitts ist, wobei das Testmittel zum Testen der Körperflüssigkeit im Testbereich dient.

8. Einrichtung nach Anspruch 1, ferner mit einem Einschneidmechanismus, der so positioniert ist, dass er in die Haut einer Person einschneidet, damit die von der Einlassöffnung des Kapillardurchgangs aufzunehmende Körperflüssigkeit austritt.

9. Einrichtung nach Anspruch 8, wobei der Einschneidmechanismus eine Stechvorrichtung umfasst, die so positioniert ist, dass an einer Stelle in der Nähe der Einlassöffnung des Kapillardurchgangs in die Haut einer Person eingestochen wird.

10. Einrichtung nach Anspruch 9, wobei die Stechvorrichtung eine ringförmige Kapillarlanzette umfasst, die ein Kapillarröhrchen und eine im Kapillarröhrchen aufgenommene Lanzette aufweist, wobei der Körper das Kapillarröhrchen aufweist und das Kapillarröhrchen den Kapillardurchgang definiert.

11. Einrichtung nach Anspruch 9, wobei der Körper einen Teststreifen umfasst.

12. Einrichtung nach Anspruch 8, ferner mit einem Ausdrückelement, das so positioniert ist, dass es die Körperflüssigkeit zur Aufnahme durch die Einlassöffnung des Kapillardurchgangs ausdrückt.

13. Einrichtung nach Anspruch 8, ferner mit einem Testbereich, der mit dem zweiten Abschnitt des Kapillardurchgangs in Verbindung steht, wobei der Testbereich eine Kapillaraffinität für die Körperflüssigkeit hat, die größer als die Kapillaraffinität des zweiten Abschnitts ist.

14. Einrichtung nach Anspruch 13, ferner mit einem Ausdrückelement, das so positioniert ist, dass es die Körperflüssigkeit zur Aufnahme durch die Einlassöffnung des Kapillardurchgangs ausdrückt.

15. Einrichtung nach Anspruch 1, wobei der Kapillardurchgang mindestens einen dritten Abschnitt aufweist, der mit dem zweiten Abschnitt in Verbindung steht und an der dem ersten Abschnitt gegenüberliegenden Seite des zweiten Abschnitts angeordnet ist, wodurch der Kapillardurchgang mehrere aufeinanderfolgende Kapillarabschnitte aufweist, wobei jeder der aufeinanderfolgenden Abschnitte des Kapillardurchgangs in der von der Einlassöffnung weg gehenden Richtung eine größere Kapillaraffinität als der vorhergehende Abschnitt hat.

16. Einrichtung nach Anspruch 15, ferner mit einem Testbereich, der mit dem letzten der aufeinanderfolgenden Abschnitte des Kapillardurchgangs in Verbindung steht und eine Kapillaraffinität für die Körperflüssigkeit hat, die größer als die Kapillaraffinität des letzten der aufeinanderfolgenden Abschnitte ist.

17. Einrichtung nach Anspruch 16, ferner mit einem Testmittel zum Testen von Körperflüssigkeit im Testbereich.

18. Einrichtung nach Anspruch 1, wobei der erste Abschnitt des Kapillardurchgangs ein vorbestimmtes Volumen hat.

19. Einrichtung nach Anspruch 18, wobei das vorbestimmte Volumen ein Volumen ist, das ausreicht, um einen nachfolgenden Abschnitt des Kapillardurchgangs zu füllen.

20. Einrichtung nach Anspruch 18, wobei das vorbestimmte Volumen ein Volumen ist, das ausreicht, um einen Test der entnommenen Körperflüssigkeit durchzuführen.

21. Einrichtung nach Anspruch 18, mit einem Testbereich, der mit dem zweiten Abschnitt des Kapillardurchgangs in Verbindung steht, wobei der Testbereich eine Kapillaraffinität für die Körperflüssigkeit hat, die größer als die Kapillaraffinität des zweiten Abschnitts ist.

22. Einrichtung nach Anspruch 21, wobei das vorbestimmte Volumen ein Volumen ist, das ausreicht, um den zweiten Abschnitt des Kapillardurchgangs zu füllen.

23. Einrichtung nach Anspruch 1, ferner mit einem Fluiddetektor, der so positioniert ist, dass er erfasst, wenn die abgenommene Körperflüssigkeit an einer Stelle im Kapillardurchgang auf der der Einlassöffnung gegenüberliegenden Seite des ersten Abschnitts des Kapillardurchgangs aufgenommen worden ist.

24. Einrichtung nach Anspruch 23, wobei der Fluiddetektor so positioniert ist, dass er erfasst, wenn die abgenommene Körperflüssigkeit im zweiten Abschnitt des Kapillardurchgangs aufgenommen worden ist.

25. Einrichtung nach Anspruch 1, ferner mit einem Fluiddetektor, der so positioniert ist, dass er erfasst, wenn der erste Abschnitt des Kapillardurchgangs gefüllt ist.

26. Einrichtung nach Anspruch 25, wobei der zweite Abschnitt des Kapillardurchgangs dem ersten Abschnitt des Kapillardurchgangs benachbart ist und eine Verbindungsstelle zu diesem hat, wobei der Fluiddetektor so positioniert ist, dass er erfasst, wenn die entnommene Körperflüssigkeit die Verbindungsstelle zwischen dem ersten Abschnitt und dem zweiten Abschnitt erreicht hat.

27. Einrichtung nach Anspruch 1, wobei der Körper eine einzige, unitäre Komponente umfasst.

28. Einrichtung nach Anspruch 1, wobei das Volumen des zweiten Abschnitts des Kapillardurchgangs größer als das Volumen des ersten Abschnitts des Kapillardurchgangs ist.

29. Einrichtung nach Anspruch 1, wobei der Kapillardurchgang mindestens einen dritten Abschnitt aufweist, der mit dem zweiten Abschnitt in Verbindung steht und an der dem ersten Abschnitt gegenüberliegenden Seite des zweiten Abschnitts angeordnet ist, wodurch der Kapillardurchgang mehrere aufeinanderfolgende Kapillarabschnitte aufweist, wobei jeder der aufeinanderfolgenden Abschnitte des Kapillardurchgangs in der von der Einlassöffnung weg gehenden Richtung eine größere Kapillaraffinität als der vorhergehende Abschnitt hat, wobei mindestens einer der aufeinanderfolgenden Kapillarabschnitte ein Volumen hat, das größer als das Volumen mindestens eines der vorhergehenden Kapillarabschnitte ist.

30. Verfahren zur Handhabung einer Probe einer Körperflüssigkeit mit folgenden Schritten:
Bereitstellen einer Entnahmeeinrichtung, die einen Körper umfasst, der einen Kapillardurchgang und eine Einlassöffnung definiert, wobei der Kapillardurchgang einen ersten Abschnitt, der mit der Einlassöffnung in Verbindung steht, und einen zweiten Abschnitt aufweist, der dem ersten Abschnitt benachbart ist und mit diesem in Verbindung steht und an der der Einlassöffnung gegenüberliegenden Seite des ersten Abschnitts angeordnet ist, wobei der erste Abschnitt des Kapillardurchgangs eine erste Kapillaraffinität für die Körperflüssigkeit hat und der zweite Abschnitt des Kapillardurchgangs eine zweite Kapillaraffinität für die Körperflüssigkeit hat, die größer als die erste Kapillaraffinität ist, und Kontaktieren der Einlassöffnung der Entnahmeeinrichtung mit einer Körperflüssigkeit, wobei das Kontaktieren das Bereitstellen einer Menge der Körperflüssigkeit umfasst, die ausreicht, um den ersten Abschnitt des Kapillardurchgangs vollständig zu füllen und den zweiten Abschnitt des Kapillardurchgangs mindestens zu kontaktieren, wodurch die Körperflüssigkeit durch die größere Affinität des zweiten Abschnitts aus dem ersten Abschnitt in den zweiten Abschnitt gesaugt wird, **dadurch gekennzeichnet, dass** die größere Kapillaraffinität auf Oberflächenbehandlung, Beschichtung, Materialauswahl oder dochtartiges Material zurückzuführen ist.

31. Verfahren nach Anspruch 30, mit dem weiteren Schritt des Testens der Körperflüssigkeit nach dem Kontaktieren.

## Revendications

1. Dispositif d'échantillonnage (11) pour acquérir un échantillon d'un fluide corporel qui comprend : un corps (14) définissant un passage capillaire (18) et une ouverture d'entrée (40), le passage capillaire incluant une première partie (39) communiquant avec l'ouverture d'entrée et une deuxième partie (41) communiquant avec la première partie et située sur le côté de la première partie opposé à l'ouverture d'entrée ; la première partie du passage capillaire ayant une première affinité capillaire pour le fluide corporel ; la deuxième partie du passage capillaire ayant une deuxième affinité capillaire pour le fluide corporel qui est plus grande que la première affinité capillaire, **caractérisé en ce que** l'affinité capillaire plus grande est due à un traitement de surface, un revêtement, une sélection de matériau ou un matériau de mèche.

2. Dispositif selon la revendication 1 dans lequel la deuxième partie du passage capillaire contient un matériau de mèche ; l'affinité capillaire du matériau de mèche étant plus grande que l'affinité capillaire de la première partie du passage capillaire.

3. Dispositif selon la revendication 1 dans lequel la surface intérieure de la première partie est revêtue avec un matériau qui est différent de celui de la surface intérieure de la deuxième partie.

4. Dispositif selon la revendication 1 et qui inclut en outre un élément d'expression positionné de manière à exprimer le fluide corporel pour une réception par l'ouverture d'entrée du passage capillaire.

5. Dispositif selon la revendication 1 et qui inclut en outre une région de test communiquant avec la deuxième partie du passage capillaire, la région de test ayant une affinité capillaire pour le fluide corporel qui est plus grande que l'affinité capillaire de la deuxième partie.

6. Dispositif selon la revendication 1 et qui inclut en outre un moyen de test pour tester le fluide corporel dans la deuxième partie du passage capillaire.

7. Dispositif selon la revendication 6 et qui inclut en outre une région de test communiquant avec la deuxième partie du passage capillaire, la région de test ayant une affinité capillaire pour le fluide corporel qui est plus grande que l'affinité capillaire de la deuxième partie, ledit moyen de test servant à tester le fluide corporel dans la région de test.

8. Dispositif selon la revendication 1 et qui inclut en outre un mécanisme d'incision positionné de manière à inciser la peau d'une personne pour produire le fluide corporel destiné à être reçu par l'ouverture d'entrée du passage capillaire.

9. Dispositif selon la revendication 8 dans lequel ledit mécanisme d'incision comprend un dispositif de piquage positionné de manière à piquer la peau de la personne en un emplacement adjacent à l'ouverture d'entrée du passage capillaire.

10. Dispositif selon la revendication 9 dans lequel ledit dispositif de piquage comprend une lancette capillaire annulaire, la lancette capillaire annulaire incluant un tube capillaire et une lancette reçue à l'intérieur du tube capillaire, ledit corps incluant le tube capillaire et le tube capillaire définissant le passage capillaire.

11. Dispositif selon la revendication 9 dans lequel ledit corps comprend une bandelette de test.

12. Dispositif selon la revendication 8 et qui inclut en outre un élément d'expression positionné de manière à exprimer le fluide corporel pour une réception par l'ouverture d'entrée du passage capillaire.

13. Dispositif selon la revendication 8 et qui inclut en outre une région de test communiquant avec la deuxième partie du passage capillaire, la région de test ayant une affinité capillaire pour le fluide corporel qui est plus grande que l'affinité capillaire de la deuxième partie.

14. Dispositif selon la revendication 13 et qui inclut en outre un élément d'expression positionné de manière à exprimer le fluide corporel pour une réception par l'ouverture d'entrée du passage capillaire.

15. Dispositif selon la revendication 1 dans lequel le passage capillaire inclut au moins une troisième partie communiquant avec la deuxième partie et située sur le côté de la deuxième partie opposé à la première partie, le passage capillaire incluant ainsi une pluralité de parties capillaires successives, chacune des parties successives du passage capillaire ayant une affinité capillaire plus grande que la partie précédente dans le sens à l'opposé de l'ouverture d'entrée.

16. Dispositif selon la revendication 15 et qui inclut une région de test communiquant avec la partie finale des parties successives du passage capillaire, la région de test ayant une affinité capillaire pour le fluide corporel qui est plus grande que l'affinité capillaire de la partie finale des parties successives.

17. Dispositif selon la revendication 16 et qui inclut en outre un moyen de test pour tester un fluide corporel dans la région de test.

18. Dispositif selon la revendication 1 dans lequel la première partie du passage capillaire a un volume prédéterminé.

19. Dispositif selon la revendication 18 dans lequel le volume prédéterminé est un volume suffisant pour remplir une partie suivante du passage capillaire.

20. Dispositif selon la revendication 18 dans lequel le volume prédéterminé est un volume suffisant pour conduire un test du fluide corporel échantillonné.

21. Dispositif selon la revendication 18 et qui inclut une région de test communiquant avec la deuxième partie du passage capillaire, la région de test ayant une affinité capillaire pour le fluide corporel qui est plus grande que l'affinité capillaire de la deuxième partie.

22. Dispositif selon la revendication 21 dans lequel le volume prédéterminé est un volume suffisant pour remplir la deuxième partie du passage capillaire.

23. Dispositif selon la revendication 1 et qui inclut en outre un détecteur de fluide positionné de manière à détecter quand le fluide corporel échantillonné a été reçu en un emplacement dans le passage capillaire sur le côté de la première partie du passage capillaire opposé à l'ouverture d'entrée.

24. Dispositif selon la revendication 23 dans lequel ledit détecteur de fluide est positionné de manière à détecter quand le fluide corporel échantillonné a été reçu à l'intérieur de la deuxième partie du passage capillaire.

25. Dispositif selon la revendication 1 et qui inclut en outre un détecteur de fluide positionné de manière à détecter quand la première partie du passage capillaire a été remplie.

26. Dispositif selon la revendication 25 dans lequel la deuxième partie du passage capillaire est adjacente à et a une jonction avec la première partie du passage capillaire, ledit détecteur de fluide étant positionné de manière à détecter quand le fluide corporel échantillonné a atteint la jonction entre la première partie et la deuxième partie.

27. Dispositif selon la revendication 1 dans lequel ledit corps comprend un composant unitaire unique.

28. Dispositif selon la revendication 1 dans lequel le volume de la deuxième partie du passage capillaire est plus grand que le volume de la première partie du passage capillaire.

29. Dispositif selon la revendication 1 dans lequel le passage capillaire inclut au moins une troisième partie communiquant avec la deuxième partie et située sur le côté de la deuxième partie opposé à la première partie, le passage capillaire incluant ainsi une pluralité de parties capillaires successives, chacune des parties successives du passage capillaire ayant une affinité capillaire plus grande que la partie précédente dans le sens à l'opposé de l'ouverture d'entrée, au moins une des parties capillaires successives ayant un volume plus grand que le volume d'au moins une des parties capillaires précédentes.

30. Procédé de manipulation d'un échantillon d'un fluide corporel qui comprend :
la prévision d'un dispositif d'échantillonnage comprenant un corps définissant un passage capillaire et une ouverture d'entrée, le passage capillaire incluant une première partie communiquant avec l'ouverture d'entrée et une deuxième partie adjacente à et communiquant avec la première partie et située sur le côté de la première partie opposé à l'ouverture d'entrée ; la première partie du passage capillaire ayant une première affinité capillaire pour le fluide corporel ; la deuxième partie du passage capillaire ayant une deuxième affinité capillaire pour le fluide corporel qui est plus grande que la première affinité capillaire ; la mise en contact de l'ouverture d'entrée du dispositif d'échantillonnage avec un fluide corporel, ladite mise en contact comprenant la prévision d'une quantité du fluide corporel suffisante pour remplir complètement la première partie du passage capillaire et pour au moins être en contact avec la deuxième partie du passage capillaire, d'où il résulte que l'affinité plus grande de la deuxième partie fait que le fluide corporel est aspiré dans la deuxième partie à partir de la première partie, **caractérisé en ce que** l'affinité capillaire plus grande est due à un traitement de surface, un revêtement, une sélection de matériau ou un matériau de mèche.

31. Procédé selon la revendication 30 et qui, après ladite mise en contact, inclut en outre un test du fluide corporel.
